# EUROPEAN PATENT SPECIFICATION

(11) **EP 3 389 810 B1**
(45) Date of publication and mention of the grant of the patent: **30.03.2022**
(21) Application number: 16816502.5
(22) Date of filing: 29.11.2016
(51) Int. Cl.: B01D 3/40, B01D 3/42, C07C 253/34, C07C 255/08

(54) **RECOVERY COLUMN CONTROL**
RÜCKGEWINNUNGSSÄULENSTEUERUNG
COMMANDE DE COLONNE DE RÉCUPÉRATION

(30) Priority: 17.12.2015 CN 201510947436
(43) Date of publication of application: 24.10.2018
(73) Proprietor: Ineos Europe AG, 1180 Rolle, Vaud (CH)
(72) Inventor: MCDONEL, Timothy, Robert, Elburn, Illinois 60119 (US); COUCH, Jay, Robert, Naperville, Illinois 60564 (US); WACHTENDORF, Paul, Trigg, Victoria, Texas 77904 (US)
(74) Representative: King, Alex
(86) International application number: PCT/US2016/063962
(87) International publication number: WO 2017/105820

(56) References cited:
- WO-A1-2009/146289
- WO-A1-2015/134718
- WO-A1-2015/183866
- GOEDECKE RALF ET AL: "Chapter 2: Verfahrensentwicklung", 19 June 2006 (2006-06-19), FLUIDVERFAHRENSTECHNIK : GRUNDLAGEN, METHODIK, TECHNIK, PRAXIS / HERAUSGEGEBEN VON, WILEY-VCH VERLAG GMBH & CO. KGAA, WEINHEIM, GERMANY, PAGE(S) 5 - 185, XP009518422, ISBN: 978-3-527-62364-8

## Description

A process is provided for operation of a recovery column for separating acrylonitrile from a mixture of acrylonitrile and acetonitrile.

### BACKGROUND

Recovery and purification systems for acrylonitrile are known, see for example U.S. Pat. Nos. 4,234,510; 3,936,360; 3,885,928; 3,433,822; and 3,399,120. Typically, propylene, ammonia and air are reacted in a vapor phase with an ammoxidation catalyst. The vaporous reactor effluent is then passed to a quench system wherein the reactor effluent is directly contacted with an aqueous quenching liquid, usually water. This quenching removes unreacted ammonia and heavy polymers. The quenched gases then proceed to an absorption column.

In the absorber, the gases are directly contacted with an absorbing liquid, again usually water. The water, acrylonitrile, acetonitrile, HCN and associated impurities leave the bottom of the absorber in an aqueous solution. Inert gases are removed from the top of the absorber. The aqueous solution then proceeds to a recovery column. This column removes acetonitrile from the aqueous solution through extractive distillation.

WO 2015/183866, for example, teaches a design for a recovery column. The column comprises a crude acetonitrile concentration zone located in the middle section of the column and defined by a baffle and a first interior facing wall of the middle section of the column.

### SUMMARY

A process for operation of a recovery column, wherein acrylonitrile is separated from a mixture of acrylonitrile and acetonitrile provided to the recovery column, the process comprising providing a temperature of 100 to 105 °C to a control tray in the recovery column prior to introduction of a feed stream into the recovery column, wherein the control tray is located in the middle section of the recovery column, and further wherein the mixture of acrylonitrile and acetonitrile is provided to a middle section of the recovery column.

The process for operation of a recovery column may include providing a temperature of 100 °C or less in a top section of the recovery column prior to introduction of a feed stream into the recovery column.

In another aspect, a process for operation of a recovery column includes providing a temperature of 100 to 105 °C to a control tray in the recovery column prior to introduction of a mixture of acrylonitrile and acetonitrile into the recovery column; providing the mixture of acrylonitrile and acetonitrile to the recovery column and contacting the mixture of acrylonitrile and acetonitrile with aqueous solvent to provide an acrylonitrile-water azeotrope; maintaining a temperature of about 55 to about 80 °C in a top portion of a top section of the recovery column; maintaining a temperature of about 65 to about 85 °C in a top portion of a middle section of the recovery column and a temperature of about 100 to about 120 °C in a bottom portion of the middle section of the recovery column; maintaining a temperature of about 105 to about 125 in a bottom portion of a bottom section of the recovery column; and separating the acrylonitrile-water azeotrope from the acetonitrile to provide an overhead stream that includes the acrylonitrile-water azeotrope, a bottoms stream, and a sidestream.

### BRIEF DESCRIPTION OF FIGURES

The above and other aspects, features and advantages of several aspects of the process will be more apparent from the following figures.
Figure 1 provides a general view of a recovery column;
Figure 2 illustrates another view of a recovery column;
Figure 3 illustrates a recovery column that includes an acetonitrile concentration zone; and
Figure 4 illustrates a recovery column temperature profile.

Corresponding reference characters indicate corresponding components throughout the several views of the drawings. Skilled artisans will appreciate that elements in the figures are illustrated for simplicity and clarity and have not necessarily been drawn to scale. For example, the dimensions of some of the elements in the figures may be exaggerated relative to other elements to help to improve understanding of various aspects. Also, common but well-understood elements that are useful or necessary in a commercially feasible aspect are often not depicted in order to facilitate a less obstructed view of these various aspects.

### DETAILED DESCRIPTION

The following description is not to be taken in a limiting sense, but is made merely for the purpose of describing the general principles of exemplary embodiments. The scope of the invention should be determined with reference to the claims.

### Recovery Column

Any type of recovery column may be utilized in connection with the present process. Several types of recovery column configurations are described herein as examples.

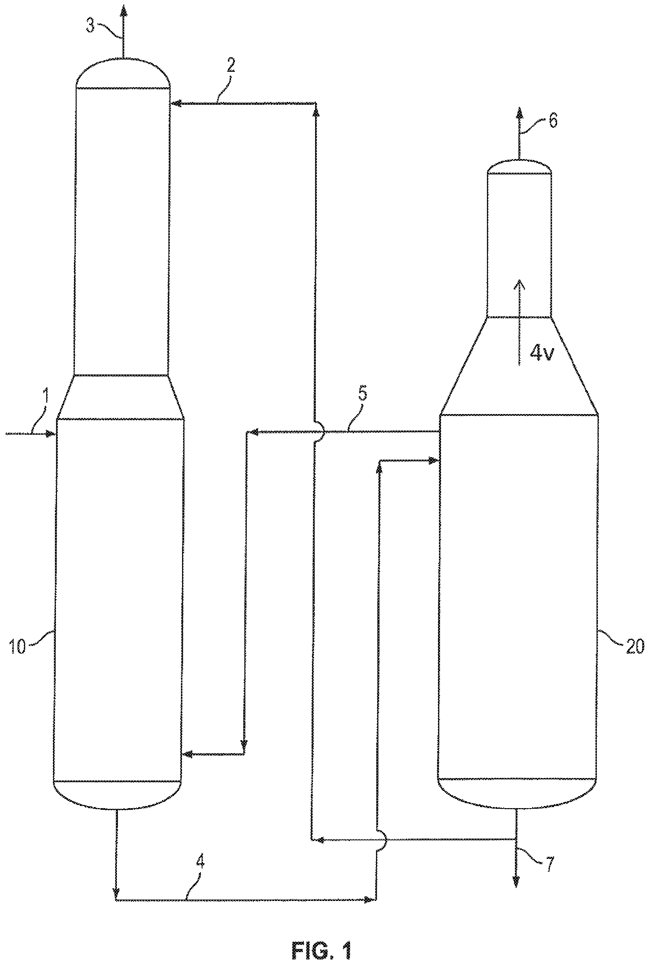

A conventional process for separation of acrylonitrile from acetonitrile is shown in FIG. 1. As shown in FIG. 1, feed stream 1 from an acrylonitrile absorber (not shown) is sent to first column 10. Feed stream 1 typically contains acrylonitrile, hydrogen cyanide (HCN), acetonitrile, and water. Water stream 2 that is substantially free of acetonitrile is recycled from at or near the bottom of second column 20 to an upper portion of first column 10 in order to facilitate the separation of acetonitrile from acrylonitrile and HCN by extractive distillation. Stream 3 containing acrylonitrile, HCN, and a portion of the water from feed 1 is removed from the top of first column 10. Liquid stream 4 containing water and acetonitrile is sent as feed from the bottom of first column 10 to second column 20. Vapor stream 5 from second column 20 is sent to first column 10 to provide the heat needed for distillation in first column 10. Vapor sidestream 4v moves up the second column 20 and includes acetonitrile. A crude acetonitrile stream 6 containing acetonitrile, water and small amounts of acrylonitrile and HCN is removed from the top of second column 20. The remaining water stream 7, which is substantially free of acrylonitrile, HCN, and acetonitrile and is not recycled as water stream 2 back to first column 10, is discharged from second column 20 at or near the bottom of second column 20.

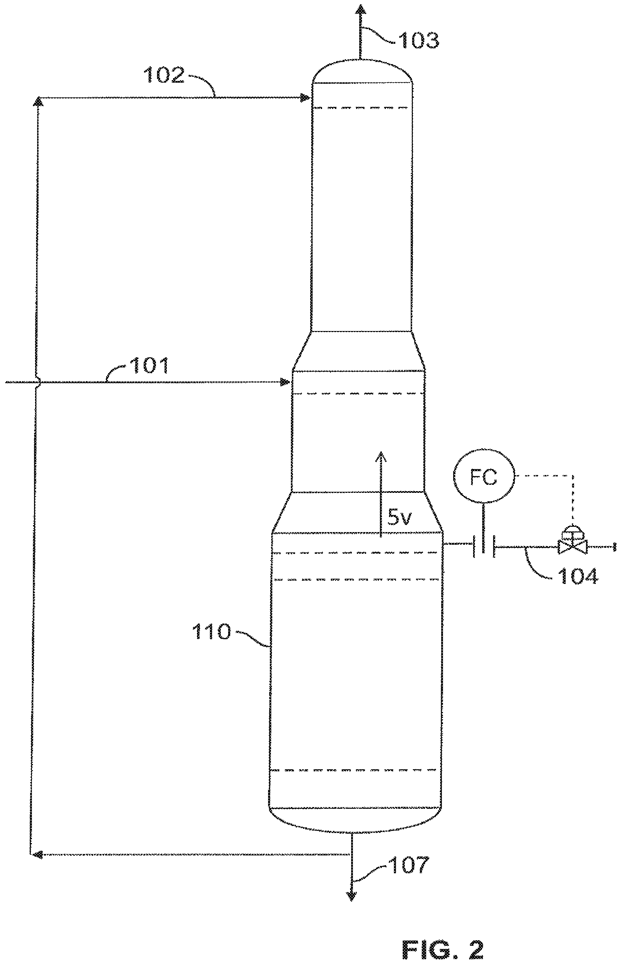

Another conventional process for separation of acrylonitrile from crude acetonitrile is shown in FIG. 2. As shown in FIG. 2, feed stream 101 from an acrylonitrile absorber (not shown) is sent to column 110. Feed stream 101 typically contains acrylonitrile, hydrogen cyanide (HCN), acetonitrile, and water. Bottoms stream 102 that is substantially free of acetonitrile is recycled from at or near the bottom of column 110 to the top of column 110 in order to facilitate the separation of acetonitrile from acrylonitrile and HCN by extractive distillation. The portion of bottoms stream 102 that is not recycled to the top of column 110 is discharged from column 110 as stream 107. Overhead stream 103 containing acrylonitrile, HCN, and a portion of the water from feed stream 101 is removed from the top of column 110. A vapor sidestream 5v moves up column 110 and a sidestream 104 (corresponding to 4v in FIG. 1) containing water and acetonitrile is removed from column 110.

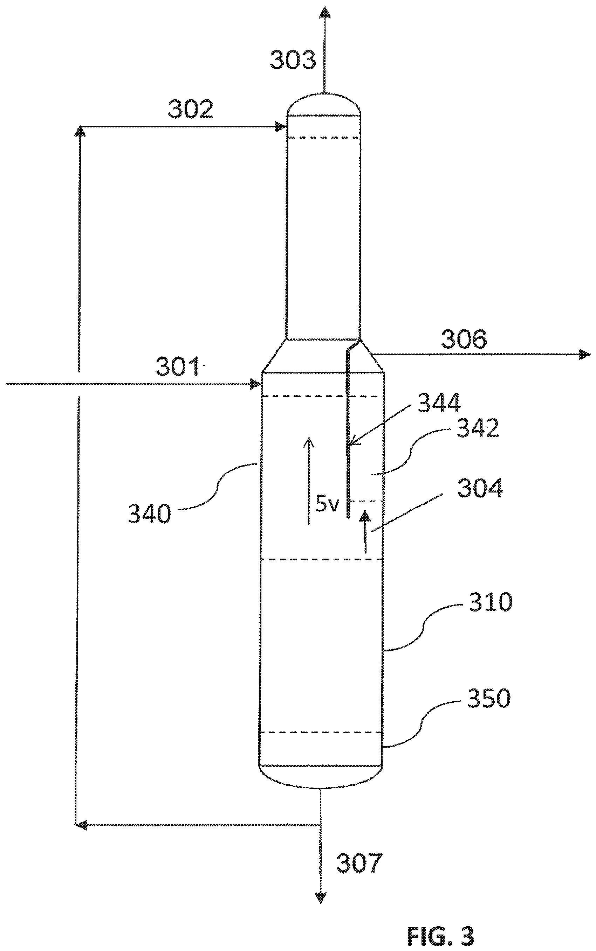

Another example of a recovery column is shown in FIG. 3. In this aspect, apparatus 300 includes column 310. Column 310 includes a top section 330, a middle section 340, and a bottom section 350. Middle section 340 of the single column 310 may be configured to receive feed stream 301. In one aspect, the column includes a top section and a middle section and a ratio of a diameter of the middle section to the top section is about 0.8 to about 1.2, in another aspect about 0.9 to about 1.1, in another aspect about 1.5 to about 2.5, in another aspect, about 1.75 to about 2.25, and in another aspect, about 1.8 to about 2. In one aspect, the column includes a middle section and a bottom section and a ratio of a diameter of the bottom section to the middle section is about 0.8 to about 1.2, in another aspect about 0.9 to about 1.1, in another aspect about 1.5 to about 2.5, in another aspect, about 1.75 to about 2.25, and in another aspect, about 1.8 to about 2. In one aspect, the column includes a top section and a bottom section and a ratio of a diameter of the bottom section to the top section is about 0.8 to about 1.2, in another aspect about 0.9 to about 1.1, in another aspect about 1.5 to about 2.5, in another aspect, about 1.75 to about 2.25, and in another aspect, about 1.8 to about 2. In one aspect, the top section, middle section and bottom section are each about 25 to about 40% of a height (tangent to tangent) of the recovery column.

As shown in FIG. 3, overhead stream 303 containing acrylonitrile, HCN and water is removed from the top of column 310. The recovery column may include a crude acetonitrile concentration zone 342. The crude acetonitrile concentration zone 342 includes internal vertical baffle 344. The crude acetonitrile concentration zone may include a plurality of trays. The trays are located at different heights of the column, and each tray includes a horizontal plane extending across a cross section of crude acetonitrile concentration zone 342. Crude acetonitrile concentration zone 342 includes an upper outlet configured to allow sidestream 306 to flow out of column 310 of crude acetonitrile concentration zone 342. Vapor may flow up either side of baffle 344 as stream 304 or as vapor stream 5v. A portion of bottoms stream 302 that is not recycled to the top of column 310 is discharged as stream 307.

The recovery column includes a top section, middle section and bottom section. In one aspect, the top, middle and bottom sections are each about 25 to about 40% of a height (tangent to tangent) of the recovery column. Each recovery column section may be further divided into portions. For example, the middle section of the recovery column includes a top portion, middle portion and bottom portion. In this aspect, the top portion, middle portion and bottom portion are each about 25 to about 40 % of a height of the middle section of the recovery column. In another aspect, the top section of the recovery column includes a top portion and a bottom portion that are each about 40 to about 60% of a height of the top section of the recovery column. In another aspect, the bottom section of the recovery column includes a top portion and a bottom portion that are each about 40 to about 60% of a height of the bottom section of the recovery column.

In another aspect, the recovery column may include about 80 to about 120 trays, and in another aspect, about 80 to about 100 trays. The plurality of trays in the recovery column includes a top section of trays, a middle section of trays, and a bottom section of trays. In this aspect, the top section of trays, middle section of trays and bottom section or trays are each about 25 to about 40% of a total number of trays in the recovery column.

The top section of trays of the recovery column includes a top portion of trays and a bottom portion of trays that are each about 40 to about 60% of a total number of trays in the top section of trays in the recovery column. The middle section of trays includes a top portion of trays, middle portion of trays and bottom portion of trays. The top portion of trays, middle portion of trays and bottom portion of trays are each about 25 to about 40% of a total number of trays of the middle section of trays of the recovery column. The bottom section of trays of the recovery column includes a top portion of trays and a bottom portion of trays that are each about 40 to about 60% of a total number of trays in the bottom section of trays in the recovery column.

### Recovery Column Operation

A feed stream provided to the recovery column typically contains acrylonitrile, hydrogen cyanide (HCN), acetonitrile, and water (shown as feed stream 1 in FIG. 1, as feed stream 101 in FIG. 2, and as feed stream 301 in FIG. 3). In this aspect, the feed stream provided to the recovery column includes about 2 to about 10 weight % acrylonitrile, in another aspect, about 3 to about 7 weight %, and in another aspect, about 4 to about 6 weight % acrylonitrile. The mixture also includes acetonitrile in an amount of about 0.1 to about 0.3 weight % acetonitrile, and in another aspect, about 0.15 to about 0.25 weight % acetonitrile. The mixture may also include other components, such as for example, acrolein and/or oxazole in smaller amounts.

Solvent water enters the recovery column at a top portion of the column (shown as water stream 2 in FIG. 1, water stream 102 in FIG. 2 and water stream 302 in FIG. 3). Solvent water which is introduced to the top of the recovery column flows down the tower across the trays, condensing and extracting acetonitrile as it travels to the bottom of the recovery column. As the liquid descends across the trays in the recovery column to the tower bottom, hot vapor from a stripper and heat from the live steam added, travel up the tower through the tray holes allowing intimate contact with the liquid on them. Heat transfer takes place between the liquid and vapor traffic and tends to move all the organics (except acetonitrile) up to the column top.

The overhead stream produced by the recovery column (shown as stream 3 in FIG. 1, stream 103 in FIG. 2, and stream 303 in FIG. 3) may include acrylonitrile, HCN, and a portion of the water from the feed stream. The recovery column may provide a vapor sidestream that includes water and acetonitrile that is substantially free of acrylonitrile and HCN (shown as stream 104 in FIG. 2, and 304 in FIG. 3). The recovery column may also provide a bottoms stream (shown as bottoms stream 2 in FIG. 1, bottoms stream 102 in FIG. 2, and bottoms stream 302 in FIG. 3) which exits from a bottom portion of the recovery column. At least a portion of the bottoms stream may be recycled back to a top portion of the recovery column. Known methods for heating the recovery column include for example, steam, steam heated reboiler and/or heat exchange with other process streams.

### Temperature Control

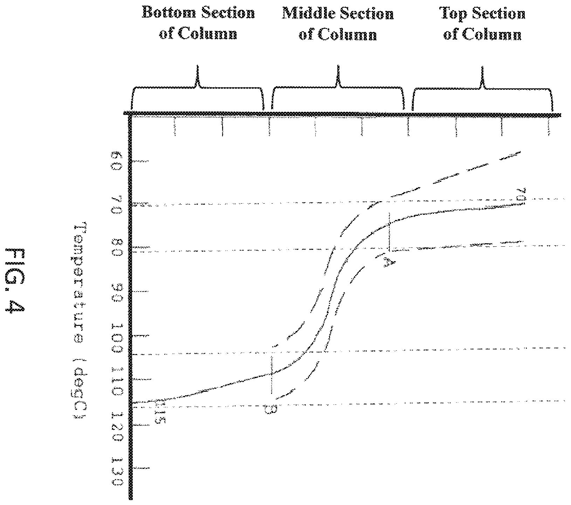

Figure 4 describes a temperature profile for recovery column operation. Proper temperature control and a temperature profile as described in Figure 4 are important for proper column operation. Variations within the temperature profile as defined by the dotted lines in Figure 4 can be effected by a combination of changes involving solvent water and feed temperatures, solvent water rate, and steam rate. Column pressure can also change the profile.

Changes above point "A" on the curve are predominantly due to variations in composition and pressure. Temperature variations above point "A" are not critical, but the overhead temperature should be kept as low as possible to minimize amounts of oxazole, acetonitrile, acetone and water going overhead. The temperature variations below point "B" are predominantly due to tray pressure changes, since the composition in this section is mostly water.

Temperature control of the recovery column includes known temperature control systems that can include reboilers and heat exchangers. In one aspect, the heat duty required to produce the necessary boil-up in the bottom of the recovery column may be provided by heat transfer in any conventional reboiling apparatus. Conventional reboilers may include some variant of a shell-and-tube exchanger. Some examples of reboiler configurations include kettle, thermosyphon, forced circulation, stab-in bundle, horizontal, vertical and falling film. In one aspect, the process includes controlling temperature by removing liquid at or near the bottom of the recovery column and exchanging the liquid in a thermosiphon reboiler. In this aspect, the effluent from the thermosiphon reboiler is returned to the recovery column. Live steam may be injected either to supplement or to replace the required heat duty of the recovery column. In another aspect, the process includes reboiling of the recovery column through two vertical thermosyphon reboilers in parallel, using pressurized steam derived from turbine exhaust.

In one aspect, recovery column temperature is maintained as follows:
the top portion of the middle section of the recovery column is maintained at a temperature of about 65 to about 85 °C, and in another aspect, about 70 to about 80 °C;
the bottom portion of the middle section of the recovery column is maintained at about 100 to about 120 °C, and in another aspect, about 105 to about 115 °C;
the top portion of the top section of the recovery column is maintained at about 55 to about 80 °C, and in another aspect, about 60 to about 75 °C;
the top portion and bottom portion of the top section of the recovery column have a temperature difference of about 0 to about 20 °C, and in another aspect, about 5 to about 15 °C;
the bottom portion of the bottom section of the recovery column is maintained at about 105 to about 125 °C, and in another aspect, about 110 to about 120 °C; and
the top portion and bottom portion of the bottom section of the recovery column have a temperature difference of about 0 to about 15 °C, and in another aspect, about 7 to about 13 °C.

In another aspect, recovery column temperature is maintained as follows:
the top portion of trays of the middle section of the recovery column is maintained at a temperature of about 65 to about 85 °C, and in another aspect, about 70 to about 80 °C;
the bottom portion of trays of the middle section of the recovery column is maintained at about 100 to about 120 °C, and in another aspect, about 105 to about 115 °C;
the top portion of trays of the top section of the recovery column is maintained at about 55 to about 80 °C, and in another aspect, about 60 to about 75 °C;
the top portion of trays and bottom portion of trays of the top section of the recovery column have a temperature difference of about 0 to about 20 °C, and in another aspect, about 5 to about 15 °C;
the bottom portion of trays of the bottom section of the recovery column is maintained at about 105 to about 125 °C, and in another aspect, about 110 to about 120 °C; and
the top portion of trays and bottom portion of trays of the bottom section of the recovery column have a temperature difference of about 0 to about 15 °C, and in another aspect, about 7 to about 13 °C.

In another aspect, temperature is controlled in the middle section of the recovery column as follows:
in one aspect, a temperature drop in the middle section of the recovery column is about 35% or greater of a temperature drop from the top tray to the bottom tray of the recovery column;
in another aspect, the temperature drop in the middle section of the recovery column is about 50% or greater of the temperature drop from the top tray to the bottom tray of the recovery column;
in another aspect, the temperature drop in the middle section of the recovery column is about 75% or greater of the temperature drop from the top tray to the bottom tray of the recovery column;
in another aspect, the temperature drop in the middle section of the recovery column is about 75% of the temperature drop from the top tray to the bottom tray of the recovery column; and
in another aspect, the temperature drop in the middle section of the recovery column is about 80% of the temperature drop from the top tray to the bottom tray of the recovery column.

Temperature control of the recovery column provides an overhead stream, bottoms stream and sidestream with the following compositions:
an overhead stream that includes the acrylonitrile-water azeotrope and about 0.05 weight percent or less acetonitrile, in another aspect, about 0.03 weight percent or less acetonitrile, and in another aspect, about 0.01 weight percent or less acetonitrile;
an overhead stream that includes about 70 weight percent to about 90 weight percent acrylonitrile, and in another aspect, about 75 to about 85 weight percent acrylonitrile;
a bottoms stream that includes about 0 to about 0.0075 weight percent acetonitrile, in another aspect, about 0.0025 to about 0.007 weight percent acetonitrile, and in another aspect, about 0.0025 to about 0.005 weight percent acetonitrile; and
a sidestream that includes about 5 to about 70 weight percent acetonitrile, in another aspect, about 5 to about 50 weight percent acetonitrile, and in another aspect, about 6 to about 12 weight percent acetonitrile.

The process for operation of a recovery column comprises providing a temperature of 100 to 105 °C to a control tray in the recovery column prior to introduction of a feed stream into the recovery column. The control tray is located in a middle section of the recovery column. The process may further include providing a temperature of 100 °C or less in a top section of the recovery column prior to introduction of a feed stream into the recovery column. In another aspect, the top section of the recovery column is 70 to 90 °C prior to introduction of the feed stream into the recovery column.

After providing the indicated temperatures, the process includes providing a mixture of acrylonitrile and acetonitrile to the middle section of the recovery column. The process includes contacting the mixture of acrylonitrile and acetonitrile with aqueous solvent to provide an acrylonitrile-water azeotrope; separating the acrylonitrile-water azeotrope from the acetonitrile to provide an overhead stream that includes the acrylonitrile-water azeotrope and about 0.05 weight percent or less acetonitrile. The process provides a bottoms stream that includes about 0 to about 0.0075 weight percent acetonitrile, and a sidestream that includes about 5 to about 70 weight % acetonitrile.

In another aspect, the process for operation of a recovery column includes providing a temperature of 100 °C or less in a top section of the recovery column prior to introduction of a feed stream into the recovery column. Preferably the top section of the recovery column is 70 to 90 °C prior to introduction of a feed stream into the recovery column.

## Claims

1. A process for operation of a recovery column, wherein acrylonitrile is separated from a mixture of acrylonitrile and acetonitrile provided to the recovery column, the process comprising providing a temperature of 100 to 105 °C to a control tray in the recovery column prior to introduction of a feed stream into the recovery column, wherein the control tray is located in the middle section of the recovery column, and further wherein the mixture of acrylonitrile and acetonitrile is provided to a middle section of the recovery column.

2. The process of claim 1 further comprising providing a temperature of 100 °C or less in a top section of the recovery column prior to introduction of the feed stream into the recovery column.

3. The process of claim 2 wherein the top section of the recovery column is 70 to 90 °C prior to introduction of the feed stream into the recovery column.

4. The process of claim 1 wherein recovery column temperature control is provided with one or more reboilers and one or more heat exchangers.

5. The process of claim 1 wherein an aqueous solvent is provided to a top section of the recovery column.

## Patentansprüche

1. Verfahren zum Betrieb einer Rückgewinnungskolonne, wobei Acrylnitril aus einem der Rückgewinnungskolonne zugeführten Gemisch von Acrylnitril und Acetonitril abgetrennt wird, wobei das Verfahren das Bereitstellen einer Temperatur von 100 bis 105 °C an einen Steuerungsboden in der Rückgewinnungssäule vor dem Eintragen eines Einsatzstoffstroms in die Rückgewinnungskolonne umfasst, wobei sich der Steuerungsboden im mittleren Teil der Rückgewinnungskolonne befindet, und ferner wobei das Gemisch von Acrylnitril und Acetonitril in einem mittleren Teil der Rückgewinnungskolonne bereitgestellt wird.

2. Verfahren nach Anspruch 1, bei dem man ferner vor dem Eintragen eines Einsatzstoffstroms in die Rückgewinnungskolonne in einem oberen Teil der Rückgewinnungskolonne eine Temperatur von 100 °C bereitstellt.

3. Verfahren nach Anspruch 2, wobei sich der obere Teil der Rückgewinnungskolonne vor dem Eintragen eines Einsatzstoffstroms in die Rückgewinnungskolonne bei 70 bis 90 °C befindet.

4. Verfahren nach Anspruch 1, wobei die Temperatursteuerung der Rückgewinnungskolonne mit einem oder mehreren Verdampfern und einem oder mehreren Wärmetauschern bereitgestellt wird.

5. Verfahren nach Anspruch 1, wobei in einem oberen Teil der Rückgewinnungskolonne ein wässriges Lösungsmittel bereitgestellt wird.

## Revendications

1. Procédé pour le fonctionnement d'une colonne de récupération, de l'acrylonitrile étant séparé d'un mélange d'acrylonitrile et acétonitrile fourni à la colonne de récupération, le procédé comprenant la fourniture d'une température de 100 à 105 °C à un plateau de contrôle dans la colonne de récupération avant l'introduction d'un flux d'alimentation dans la colonne de récupération, le plateau de contrôle étant situé dans la section centrale de la colonne de récupération, et en outre le mélange d'acrylonitrile et acétonitrile étant fourni à une section centrale de la colonne de récupération.

2. Procédé selon la revendication 1 comprenant en outre la fourniture la fourniture d'une température de 100 °C ou moins dans une section supérieure de la colonne de récupération avant l'introduction du flux d'alimentation dans la colonne de récupération.

3. Procédé selon la revendication 2, la section supérieure de la colonne de récupération étant à une température de 70 à 90 °C avant l'introduction du flux d'alimentation dans la colonne de récupération.

4. Procédé selon la revendication 1, le contrôle de la température de la colonne de récupération étant fourni avec un ou plusieurs rebouilleurs et un ou plusieurs échangeurs de chaleur.

5. Procédé selon la revendication 1, un solvant aqueux étant fourni à une section supérieure de la colonne de récupération.
